# EUROPEAN PATENT APPLICATION

(11) **EP 2 193 772 A1**
(43) Date of publication of application: **09.06.2010**
(21) Application number: 08778072.2
(22) Date of filing: 11.07.2008
(51) Int. Cl.: A61F 13/15, A61F 13/49, A61F 13/494

(54) **ABSORBENT ARTICLE**

(30) Priority: 28.09.2007 JP 2007255992
(71) Applicant: Unicharm Corporation, Ehime 799-0111 (JP)
(72) Inventor: MINATO, Hironao, Kanonji-shi Kagawa 769-1602 (JP); NAKAJIMA, Kaiyo, Kanonji-shi Kagawa 769-1602 (JP); KAMIYAMA, Ryuichi, Kanonji-shi Kagawa 769-1602 (JP)
(74) Representative: Stark, Gordon Drummond
(86) International application number: PCT/JP2008/062556
(87) International publication number: WO 2009/041145

(57) **Abstract**

The present invention aims to provide an improved absorbent article which alleviates any possible skin irritation experienced by a wearer when a barrier sheet comes in contact with the wearer' s skin, and which prevents pressure marks from being left on the wearer's skin due to contact between the barrier sheet and the diaper wearer's skin.

A liquid-absorbent chassis 2 is provided on an inner surface 100 thereof which faces the wearer's skin, by means of a barrier sheet 8 which comprises front and rear passage openings 27, 28 defined by lateral portions 34, 35 and a middle portion 26, and barrier sheet elastic members 33 functioning to space the barrier sheet 8 upwardly from the liquid-absorbent chassis 2. The barrier sheet 8 includes first adhesion sites 40, 41 at which front and rear end portions 24, 25 are bonded to the barrier sheet 8, second adhesion sites 42 at which convex segments 36 of the barrier sheet elastic members 33 are bonded to the barrier sheet 8 and third adhesion sites 43 defined between respective pairs of first adhesion sites 40, 41 and a pair of the second adhesion sites 42 and at which segments of the barrier sheet elastic members 33 occupying these sites 43 are bonded to the barrier sheet 8. The adhesive forming the second and third adhesion sites 42, 43 has a basis weight lower than the basis weight of the adhesive forming the first adhesion sites 40, 41.

## Description

### TECHNICAL FIELD

The present invention relates to an absorbent article and particularly to an absorbent article such as a disposable diaper, toilet training pants or incontinent briefs.

### RELATED ART

Disposable diapers which have a specific construction adapted to protect the diaper wearer' s skin from contamination with body waste are well known, for example, from JP 2007-105302A.
PATENT DOCUMENT 1: JP 2007-105302A

### DISCLOSURE OF THE INVENTION

### PROBLEM TO BE SOLVED BY THE INVENTION

JP 2007-105302A discloses a pants-type disposable diaper. This diaper comprises a pants-shaped cover sheet and a liquid-absorbent structure provided on the inner side of the cover-sheet wherein the liquid-absorbent structure is provided thereon with a skin-contact sheet. The skin-contact sheet has a front passage opening, a rear passage opening, a middle portion defined between these two passage openings, and elastic members attached thereto under tension so as to extend in a longitudinal direction. Specifically, these elastic members are attached to the skin-contact sheet along respective inner edges defining the front and rear passage openings so that the skin-contact sheet may be spaced upward from the liquid absorbent structure under contraction of the elastic members and, in consequence, the middle region of the skin-contact sheet may come in contact with the diaper wearer' s skin. In this way, the liquid-absorbent structure is prevented from coming in direct contact with the wearer's skin.

Under contraction of the elastic members, the skin-contact sheet is formed on its inner side facing the wearer's skin with gathers which come in contact with the wearer's skin. In order that the elastic members can be attached under tension to the skin-contact sheet, at least longitudinally opposite end segments thereof must be permanently bonded to the sheet, or there will be a possibility that the elastic members may fall off from the sheet. According to the disclosure of JP 2007-105302, this problem is dealt with by coating the skin-contact sheet over its entire area with suitable adhesive of basis weight at as high a level as is required to reliably affix the longitudinally opposite end segments of the elastic members. Thus the skin-contact sheet is coated over its entire area with an excessive quantity of the adhesive, normally, hot melt adhesive in order to prevent the elastic members from unintentionally falling off. In consequence, due to the adhesive covering, the stiffness of the sheet as a whole increases unacceptably and in turn the occurrence of skin irritation correspondingly increases. In particular, the portion of the skin-contact sheet formed with gathers may often leave on the wearer' s skin, such as a pressure mark, due to such skin irritation.

In view of the problem as has been described above, the present invention provides an absorbent article improved so as to alleviate any possible skin irritation experienced by the diaper wearer when a barrier sheet comes in contact with the wearer's skin and which prevents the above-described pressure mark from being left on the wearer's skin due to contact of the barrier sheet with the diaper wearer's skin.

### MEASURE TO SOLVE THE PROBLEM

According to the present invention, there is provided an improved absorbent article comprising a liquid-absorbent chassis having a longitudinal direction, a transverse direction, a side facing the article wearer's body, a side facing the wearer' s garment, a front waist region, a rear waist region, a crotch region extending between the front and rear waist region and a liquid-absorbent structure provided in the crotch region, and a barrier sheet provided on the side facing the article wearer's body of the liquid-absorbent chassis which is adapted to be spaced from the liquid-absorbent chassis wherein the barrier sheet includes barrier sheet elastic members attached under tension thereto and extending in the longitudinal direction and a space which is formed between the barrier sheet and the liquid-absorbent structure as the liquid-absorbent structure is bowed in the longitudinal direction.

The improvement according to the present invention is **characterized in that** the barrier sheet comprises a pair of lateral portions opposed to and spaced from each other in the transverse direction, a middle portion connecting the lateral portions to each other, front and rear passage openings defined by the lateral portions and a middle portion through which body waste can be guided into a space, and wherein the lateral portions are attached at front and rear end portions thereof to the front and rear waist regions, the barrier sheet elastic members being attached to the lateral portions and extending along the passage openings, and the barrier sheet having first adhesion sites at which front and rear end segments of the barrier sheet elastic members are anchored to the barrier sheet and middle adhesion sites at which middle segments of the barrier sheet elastic members extending between the first adhesion sites are anchored to the barrier sheet, and wherein the basis weight of adhesive is lower in the middle adhesion sites than in the first adhesion sites.

According to one preferred embodiment, the barrier sheet elastic members extend from the front end portions of the lateral portions to the rear end portions and describe convex segments bowing toward a longitudinal center line bisecting the barrier sheet in the transverse direction, and the middle adhesion sites comprise second adhesion sites at which the convex segments are anchored to the barrier sheet, and third adhesion sites at which segments of the barrier sheet elastic members which extend between the first adhesion sites and the second adhesion sites are anchored to the barrier sheet.

According to another preferred embodiment, each of the barrier sheet elastic members extends from the front end portion of one of the lateral portions to the rear end portion of the other lateral portions, so that the barrier sheet elastic members intersect with each other in the middle portion, and wherein the middle portion includes a second adhesion site at which the intersecting segments are anchored to the barrier sheet, and a third adhesion site at which segments of the barrier sheet elastic members extending between the first adhesion sites and the second adhesion sites are anchored on the barrier sheet.

According to still further preferred embodiment, the adhesive of the third adhesion sites has a basis weight lower than the basis weight of the adhesive in the second adhesion sites.

According to further another preferred embodiment, the barrier sheet is coated at the first adhesion sites and the second adhesion sites over the entire areas thereof with the adhesive and intermittently coated with the adhesive at the third adhesion sites.

### EFFECT OF THE INVENTION

According to the present invention, the barrier sheet elastic members extend in the longitudinal direction across the lateral portions of the barrier sheet, and the longitudinally opposite end segments thereof are anchored on the barrier sheet at the first adhesion sites formed by an adhesive having a basis weight higher than that used at the other adhesion sites. In this way, the barrier sheet elastic members are reliably protected from becoming unstuck or falling off. By ensuring that the barrier sheet elastic members are protected from falling off, it is possible to reduce the basis weight of the adhesive at the other adhesion sites, thereby reducing the stiffness of the barrier sheet so that undesired skin irritation possibly experienced by the wearer can be correspondingly alleviated. With stiffness of the barrier sheet reduced in this manner, the gathers created in the barrier sheet under contraction of the barrier sheet elastic members are softened. As a result, the skin irritation possibly experienced by the wearer can be alleviated and pain and/or pressure marks caused by the contact of the barrier sheet with the wearer's skin can be prevented.

### BRIEF DESCRIPTION OF THE DRAWINGS

[FIG. 1] Fig. 1 is a perspective view of a first embodiment of the present invention.
[FIG. 2] Fig. 2 is a sectional view taken along the line II-II in Fig. 1.
[FIG. 3] Fig. 3 is a plan view showing the diaper of Fig. 1 in a flat opened out configuration.
[FIG. 4] Fig. 4 is an exploded perspective view of the plan view of the diaper of Fig. 3.
[FIG. 5] Fig. 5 is an exploded perspective view of the barrier sheet according to the first embodiment.
[FIG. 6] Fig. 6 is a scale-enlarged plan view of the second sheet forming the barrier sheet according to the second embodiment.
[FIG. 7] Fig. 7 is a view similar to Fig. 6, illustrating an embodiment which varies from that illustrated by Fig. 6.
[FIG. 8] Fig. 8 is a scale-enlarged plan view of the first sheet forming the barrier sheet according to the third embodiment.
[FIG. 9] Fig. 9 is a scale-enlarged plan view of the second sheet forming the barrier sheet according to the fourth embodiment.

### IDENTIFICATION OF REFERENCE NUMERALS USED IN THE DRAWINGS

- 1: diaper
- 2: liquid-absorbent chassis
- 3: front waist region
- 4: rear waist region
- 5: crotch region
- 7: liquid-absorbent structure
- 8: barrier sheet
- 24: front end portion
- 25: rear end portion
- 26: middle portion
- 27: front passage opening
- 28: rear passage opening
- 33: barrier sheet elastic member
- 34: lateral portion
- 35: lateral portion
- 36: convex segments
- 37: front end portion
- 38: rear end portion
- 40: first adhesion sites
- 41: first adhesion sites
- 42: second adhesion sites
- 43: third adhesion sites

### DESCRIPTION OF THE BEST MODE FOR WORKING OF THE INVENTION

### <Example 1 - First Embodiment>

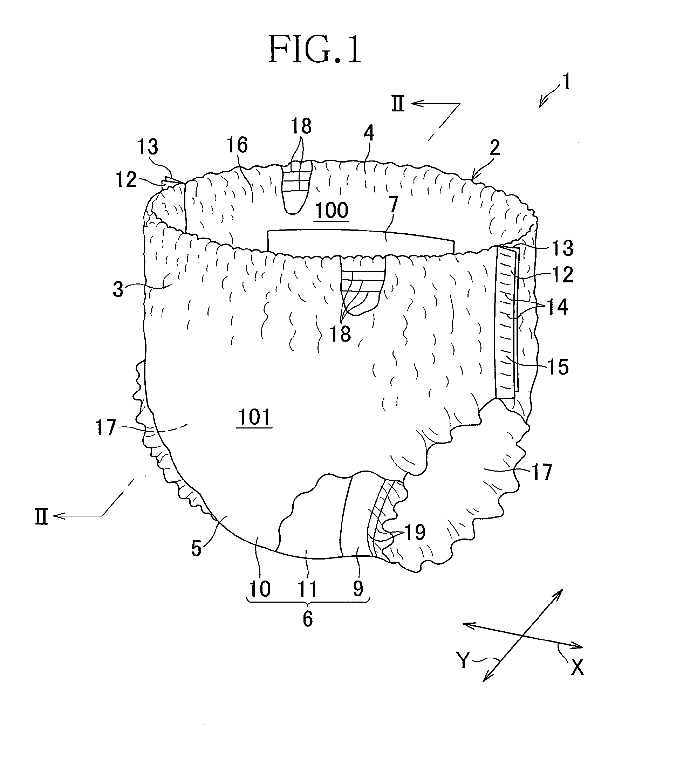

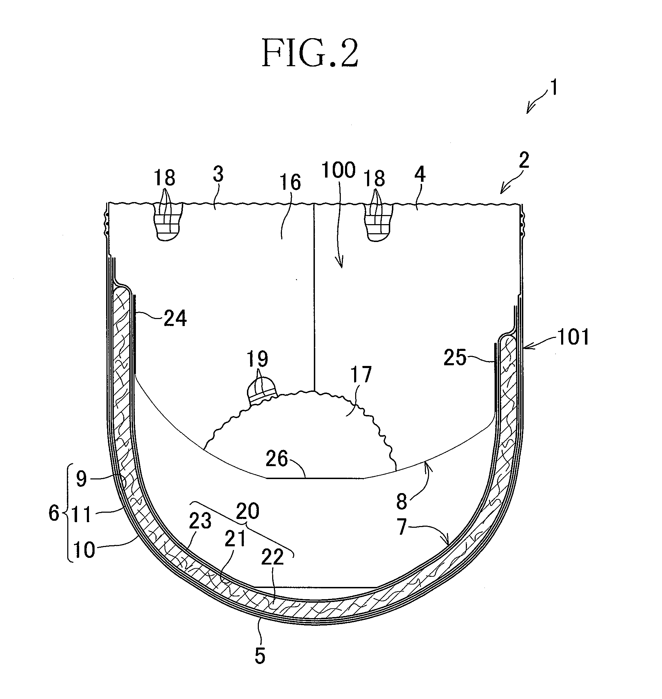

Figs. 1 through 5 illustrate a first embodiment of the invention. Fig. 1 is a partially cutaway perspective view of a disposable diaper as a typical example of the absorbent article and Fig. 2 is a sectional view taken along the line II-II in Fig. 1. Fig. 1 shows the diaper 1 in the schematic configuration assumed when it is worn on the wearer's body, wherein a transverse direction and a longitudinal direction of the diaper 1 are indicated by arrows X and Y, respectively.

Referring to Fig. 1, the diaper 1 includes a liquid-absorbent chassis 2. The liquid-absorbent chassis 2 has an inner surface 100 facing the wearer's body, an outer surface 101 facing the wearer's garment, a front waist region 3, a rear waist region 4 and a crotch region 5 extending between the front and rear waist regions 3, 4. From a different viewpoint, the diaper 1 comprises a pant-shaped cover sheet 6, a liquid-absorbent structure 7 provided on the inside of the cover sheet 6, i.e., on the inner surface 100 facing the wearer's body, and a barrier sheet 8 provided on the inside of the liquid-absorbent structure 7. The cover sheet 6 comprises, in turn, an inner sheet 9, an outer sheet 10 and a liquid-impervious sheet 11 sandwiched between these two sheets 9, 10, defining together the front waist region 3, the rear waist region 4 and the crotch region 5. The front and rear waist regions 3, 4 are put flat and joined together along respectively opposed waist side edges 12, 13 at a plurality of joins 14 arranged intermittently along these waist side edges 12, 13 to form seams 15.

The waist side edges 12, 13 are joined together at the joins 14 and thereby a waist-opening 16 is formed in a region surrounded by the front and rear waist regions 3, 4, and at the same time a pair of leg-openings 17, respectively surrounded by the joins 14 and the crotch region 5, are formed. A plurality of waist elastic members 18 extend along a peripheral edge of the waist-opening 16 and a plurality of leg elastic members 19 extend along peripheral edges of the respective leg-openings 17. These elastic members 18, 19 are sandwiched between the inner sheet 9 and the outer sheet 10 and bonded under tension to at least one of these sheets 9, 10 by means of adhesive (not shown).

Referring to Fig. 2, the liquid-absorbent structure 7 extends in the longitudinal direction Y between the front and rear waist regions 3, 4, but the liquid-absorbent structure 7 effectively functions, so far as it is present, at least in the crotch region 5. This liquid-absorbent structure 7 includes a liquid-absorbent panel 20 extending on the inner surface of the cover sheet 6. The liquid-absorbent panel 20 comprises a liquid-absorbent core 22 wrapped with a liquid-absorbent and -spreadable sheet 21, such as tissue paper, and an inner sheet 23 adapted to cover at least an area of the upper surface of the core 22 which faces the wearer's skin. The outer surface (bottom surface) of the panel 20 opposed to the inner sheet 23 is covered with the liquid-impervious sheet 11 by the intermediary of the outer sheet 10. The liquid-impervious sheet 11 may sometimes be directly bonded to the bottom surface of the liquid-absorbent panel 20.

In the front and rear waist regions 3, 4, at least a front end portion 24 and a rear end portion 25 of the barrier sheet 8 are bonded to the inner sheet 23 and, if it is necessary, along lateral portions 44, 45 also. The manner in which the barrier sheet 8 is attached is not limited to the illustrated embodiment and, for example, in the case of the diaper provided with the leak-barrier cuffs, it is also possible to bond the barrier sheet 8 to these leak-barrier cuffs.

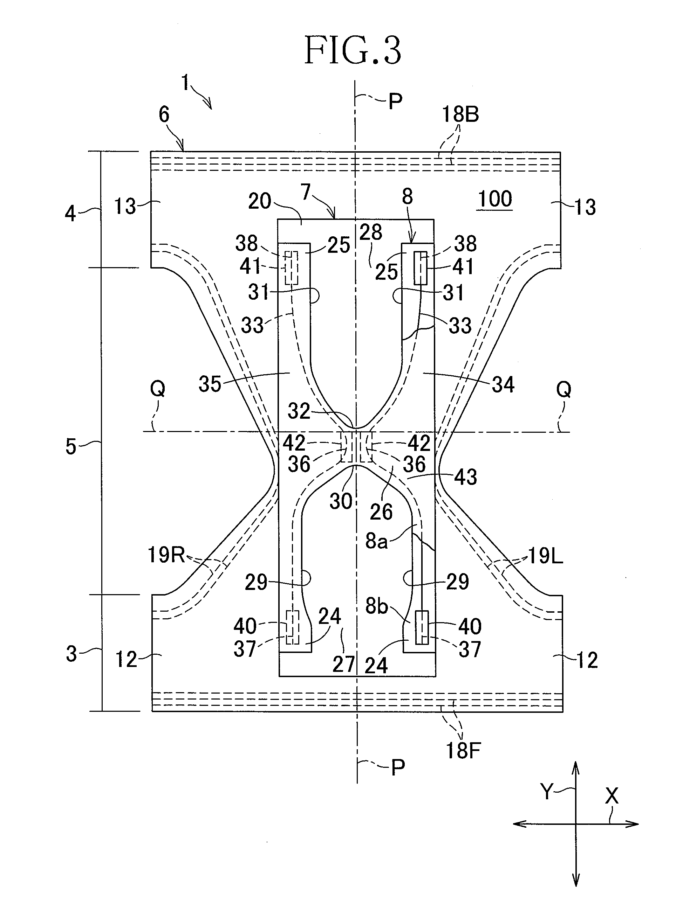

Fig. 3 is a plan view of the diaper 1 obtained by extending the diaper 1 in the longitudinal direction Y as well as in the transverse direction X, after ripping the front and rear waist regions 3, 4 from each other along each column of the joins 14. In Fig. 3, the elastic members are held in a stretched state so that the diaper 1 may remain in a flattened configuration. The expression "left and right" as used in the following description mean to be left and right relative to the wearer of the diaper 1. The diaper 1 has a longitudinal center line P-P bisecting a dimension of the diaper 1 in the transverse direction X and a transverse center line Q-Q bisecting a dimension of the diaper 1 in the longitudinal direction X. The diaper 1 is bilaterally-symmetric about the longitudinal center line P-P.

The cover sheet 6 is formed substantially in a general hourglass-shape and the liquid-absorbent panel 20 is formed in a rectangular shape. The waist elastic members 18 provided on the cover sheet 6 comprise front and rear waist-surrounding elastic members 18F, 18B as indicated by broken lines while the leg elastic members 19 comprise left and right leg elastic members 19L, 19R as indicated also by broken lines.

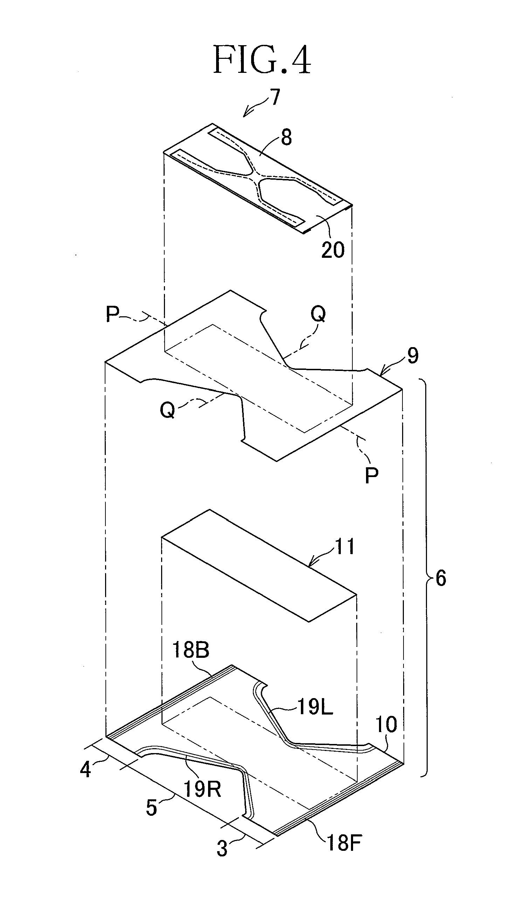

As will be apparent from Fig. 4 which is an exploded perspective view of the diaper 1, the liquid-absorbent panel 20 is laminated on the cover sheet 6. The outer sheet 10 constituting the cover sheet 6 is formed from a fibrous nonwoven fabric or moisture-pervious plastic film of which the inner surface (i.e., upper surface as viewed in Fig. 4) facing the wearer's skin is provided with the waist elastic members 18F, 18B and the leg elastic members 19L, 19R attached under tension thereto by means of hot melt adhesive (not shown). The liquid-impervious sheet 11 formed from a liquid-impervious and moisture-pervious plastic film is attached to the inner surface of the outer sheet 10 by means of adhesion or welding. As has previously been described, this liquid-impervious sheet 11 may be directly bonded to the bottom surface of the liquid-absorbent panel 20 and, in this case, the liquid-impervious sheet 11 preferably has at least an area sufficient to cover the bottom surface of the liquid-absorbent core 22 from the viewpoint of the liquid-impervious effect.

The liquid-impervious sheet 11 is provided on its inner surface with the inner sheet 9 formed from a fibrous nonwoven fabric or moisture-pervious plastic film and having the shape as well as the size same as those of the outer sheet 10 bonded thereto by means of adhesion or welding. The inner sheet 9 is provided, in turn, on its inner surface with the liquid-absorbent panel 20 bonded thereto. The liquid-absorbent panel 20 is intermittently coated on its substantially entire outer surface (i.e., bottom surface as viewed in Fig. 4) with hot melt adhesive (not shown) by means of which the liquid-absorbent panel 20 is bonded to the inner sheet 9. The liquid-absorbent panel 20 is provided on its inner surface with the barrier sheet 8.

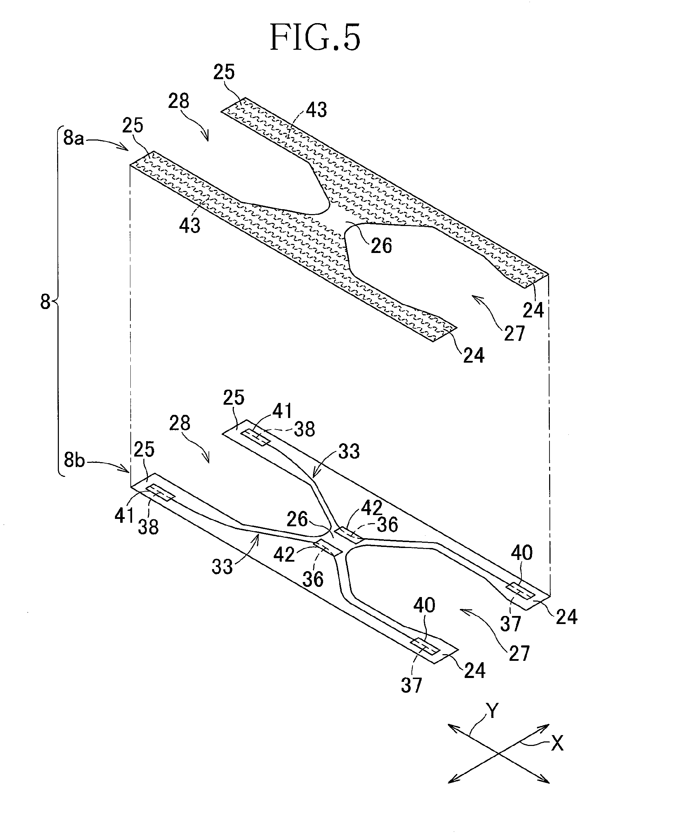

Referring to Figs. 3 and 5, the barrier sheet 8 comprises a first sheet 8a facing the wearer's skin and a second sheet 8b facing away from the wearer's skin, both bonded together. Each of these first and second sheets 8a, 8b is formed from a sheet piece of nonwoven fabric or liquid-pervious plastic film or a laminate thereof and preferably liquid-impervious.

As will be apparent from Fig. 3, the barrier sheet 8 is formed by a sheet material such as a fibrous nonwoven fabric, moisture-pervious plastic film or laminate thereof and preferably liquid-impervious. The barrier sheet 8 has lateral portions 44, 45 opposed to and spaced from each other in the transverse direction X and a middle portion 26 extending between the lateral portions 44, 45 wherein the middle portion 26 is present in the crotch region 5. The lateral portions 44, 45 cooperate with the middle portion 26 to define passage openings, specifically, a substantially U-shaped front passage opening 27 extending from the middle portion 26 toward the front waist region 3 and a substantially U-shaped rear passage opening 28 extending from the middle potion 26 toward the rear waist region 4. The front passage opening 27 is defined by two inner side edges 29 of the respective lateral portions 34, 35 and a curved closure edge 30 connecting the inner side edges 29L, 29R with each other and the rear passage opening 28 is defined by two inner side edges 31 of the lateral portions and a curved closure edge 32 connecting these inner side edges with each other. The front passage opening 27 and the rear passage opening 28 respectively extend from the front and rear end portions 24, 25 of the barrier sheet 8 toward the middle portion 26 so that the region of the sheet allocated to come in direct contact with the wearer's skin may be made minimized and undesired stuffiness due to sweat or the other secretion may be prevented.

When the diaper 1 is put on the wearer's body, the relative position of the diaper 1 on the wearer's body should be adjusted so that the front passage opening 27 is opposed to the wearer's external genitals, the rear passage opening 28 is opposed to the wearer's anus and the middle portion 26 comes in contact with the wearer's skin in a region defined between the external genital and the anus. With the diaper 1 put on the wearer's body in this manner, preferably the closure edge 30 of the front passage opening 27 is placed forward from the transverse center line Q-Q and the closure edge 32 of the rear passage opening 28 is just on or in the vicinity of the transverse center line Q-Q. With the diaper put on the wearer's body in the manner as has been described above, the liquid-absorbent structure 7 is bowed in the longitudinal direction Y and simultaneously the above-described sheet region rises up as the barrier sheet elastic members 34L, 34R contract so as to create a space between the barrier sheet 8 and the liquid-absorbent structure 7. In this way, a free region including the middle region of the barrier sheet 8 which is opposed to the inner sheet 23 is spaced from this inner sheet 23 in the thickness direction of the diaper 1 (See Fig. 2).
While not shown, the barrier sheet 8 may have the same shape and size as the inner sheet 9 constituting a part of the cover sheet 6. It is also possible to close the front and rear passage openings 27, 28 in the front and rear waist regions 3, 4, respectively.

As will be apparent from Fig. 5, the barrier sheet elastic members 33 are sandwiched between the first and second sheets 8a, 8b and attached under tension thereto wherein each of the right and left side elastic members on the right and left side preferably comprises a single rubber thread extending in the longitudinal direction Y substantially along the associated inner side edge 29, 31 of the lateral portions 34, 35 inclusive of the front and rear passage openings 27, 28. The barrier sheet elastic members 33 include substantially straight segments in the vicinities of the respective inner side edges 29, 31 of the front and rear passage openings 27, 28. In the middle portion 26, the barrier sheet elastic members 33 include convex segments 36 extending along the closure edges 30, 32 toward the longitudinal center line P-P.

Front and rear end portions 37, 38 of the barrier sheet elastic members 33 are bonded to the front and rear end portions 24, 25 of the barrier sheet 8 as viewed in the longitudinal direction Y, respectively, by means of hot melt adhesive to define first adhesion sites 40, 41. Between the respective pairs of the first adhesion sites 40, 41 as viewed in the longitudinal direction Y, the respective convex segments 36 are bonded to the middle portion 26 by means of hot melt adhesive to form second adhesion sites 42. Between respective pairs of the first adhesion sites 40, 41 and a pair of the second adhesion sites 42 as viewed in the longitudinal direction Y, the barrier sheet 8 is provided with a pair of third adhesion sites 43, respectively, serving not only to fix the corresponding segments of the elastic members 33 to the barrier sheet 8 but also to keep the first and second sheets 8a, 8b put flat and bonded together. The hot melt adhesive used to form these adhesion sites may be selected from those widely used in this field of art, commonly referred to as rubber-based hot melt adhesive, such as styrene-butadiene-styrene block copolymer (SBS), styrene-isoprene-styrene block copolymer (SIS), styrene-ethylene-butylene-styrene block copolymer (SEBS) and styrene-ethylene-propylene-styrene block copolymer (SEPS).

The first adhesion sites 40, 41 are provided principally for the purpose of preventing the barrier sheet elastic members 33 from falling off and therefore must be adequately retentive. To this end, in relation to the first adhesion sites 40, 41, the hot melt adhesive has a sufficiently high basis weight to achieve the desired adhesive strength. Specifically, the first adhesion sites 40, 41 of the second sheet 8b are coated upon their entire areas, each 10 mm in the width and 30 mm in the length, with the hot melt adhesive so as to have a basis weight of 30 g/m².

The second adhesion sites 42 serve to anchor the barrier sheet elastic members 33 which would otherwise be biased to move outward in the transverse direction X. It is not required for the second adhesion sites 42 to have an adhesive strength as high as that required for the first adhesion sites 40, 41. Specifically, the second adhesive sites 42 of the second sheet 8b are coated on the respective entire areas, each 10 mm in width and 30 mm in length, with the hot melt adhesive so as to have a basis weight of 10 g/m² which is substantially lower than in the first adhesion sites 40, 41.
It is possible to coat the second adhesion sites 42 with the hot melt adhesive in such a manner that the hot melt adhesive moves inward beyond the inner edges defining the front and rear passage openings 27, 28 which are still not cut away. In this case, after the portions of the sheet 8 which will define the front and rear passage openings 27, 28 have been cut away, the hot melt adhesive is present also along the inner edges of the front and rear passage openings 27, 28.

The third adhesion sites 43 serve to prevent the barrier sheet elastic members 33 from sticking out the lateral portions 34, 35 or the front and rear passage openings 27, 28 and also to bond the first and second sheets 8a, 8b to each other. The third adhesions sites 43 may have an adhesive strength lower than those of the first and second adhesion sites 40, 41 and 42. In other words, the basis weight of the hot melt adhesive forming the third adhesion sites 43 may be lower than those of the first and second adhesion sites 40, 41 and 42. Specifically, the first sheet 8a is coated with the hot melt adhesive for the third adhesion sites 43 so as to form a wave pattern. The hot melt adhesive forming these third adhesion sites 43 may have a basis weight of 5 g/m² lower than those of the hot melt adhesive forming the first and second adhesion sites 40, 41 and 42.
The basis weight of the hot melt adhesive forming the respective adhesion sites may be adjusted in this manner to ensure that the first adhesion sites 40, 41 have the highest basis weight, that the second adhesion sites 42 have a basis weight lower than that of the first adhesion sites 40, 41 and that the third adhesion sites 43 have a basis weight lower than that of the second adhesion sites 42.
According to this embodiment, the barrier sheet 8 is coated substantially over its entire surface with the hot melt adhesive at a basis weight as low as the basis weight of the third adhesion sites 43, in a wave pattern. Substantially the entire area of a region of the barrier sheet 8 is coated with the hot melt adhesive and occupied by the barrier sheet elastic members 33 partially defines the third adhesion sites 43.

The surfaces of the first and second sheets 8a, 8b coated with the hot melt adhesive are put flat and bonded together to obtain the barrier sheet 8. More specifically, the first sheet 8a is coated with the hot melt adhesive defining the third adhesion sites 43, while the second sheet 8b is coated with the hot melt adhesive defining the first adhesion sites 40, 41 and the second adhesion sites 42. The longitudinally opposite end portions 37, 38 of the barrier sheet elastic members 33 are placed on the first adhesion sites 40, 41 while the convex segments 36 of the barrier sheet elastic members 33 are placed on the second adhesion sites 42 of the second sheet 8b and thereafter the first sheet 8a is put flat and bonded together with the second sheet 8b.

The first and second sheets 8a, 8b are put flat and bonded together after the respective sheets 8a, 8b have been coated with the hot melt adhesive, as has been described above. Therefore, the first and second sheets 8a, 8b can be processed through the use of coater units exclusively used for the respective sheets 8a, 8b so that a pattern of coating as well as a basis weight of coating can be independently regulated for the respective sheets 8a, 8b.

In the course of coating, the hot melt adhesive forming the first and second adhesion sites 40, 41 and 42 has a melt viscosity of 70000 mPas at a temperature of 140°C, while the hot melt adhesive forming the third adhesion sites 43 has a melt viscosity of 11000 mPas at a temperature of 140°C. In this way, the melt viscosity is adapted for desired coating characteristic and a cohesion force is regulated so as to be higher in the first adhesion sites 40, 41 and the second adhesion sites 42, than in the third adhesion sites 43. By increasing the cohesion force, an anchoring effect for the barrier sheet elastic members 33 can be correspondingly increased and a quantity of the hot melt adhesive used to form the first adhesion sites 40, 41 and the second adhesion sites 42 can be correspondingly reduced. As will be appreciated by those skilled in the art, it is not essential to coat the hot melt adhesive at different melt viscosities to form the first adhesion sites 40, 41, the second adhesion sites 42 and the third adhesion sites 43, but it is also possible to coat the hot melt adhesive at one and the same melt viscosity.

The hot melt adhesive used to form the first adhesion sites 40, 41 is regulated to have the highest basis weight sufficient to anchor the longitudinal opposite end portions 37, 38 of the barrier sheet elastic members 33 in a reliable manner to avoid the elastic members 33 falling off from the diaper. At the second adhesion sites 42 and the third adhesion sites 43, the hot melt adhesive has the basis weight lower than that at the first adhesion sites 40, 41 and therefore the barrier sheet can be maintained correspondingly soft. Consequentially, undesired irritation on the wearer's skin, particularly around the wearer's genital organs and buttocks can be effectively alleviated.

The first adhesion sites 40, 41 are located to overlap with the longitudinally opposite end portions 37, 38 of the barrier sheet elastic members 33. Contraction of the barrier sheet elastic members 33 is constrained along the longitudinally opposite end portions 37, 38 due to the high cohesion force of the hot melt adhesive used in these adhesion sites and the number of gathers formed along these segments is also restricted. In consequence, the skin irritation can be alleviated. The first adhesion sites 40, 41 are required only to anchor the longitudinally opposite end portions 37, 38 of the barrier sheet elastic members 33 and each of the first adhesion sites 40, 41 may have a relatively small area. In other words, there is no anxiety that these adhesion sites 40, 41 might significantly irritate the wearer's skin even when the basis weight of these sites is increased. In the second adhesion sites 42, the hot melt adhesive has the highest basis weight next to the first adhesion sites 40, 41 sufficient to anchor the convex segments 36 of the barrier sheet elastic members 33. Furthermore, the second adhesion sites 42 allow the barrier sheet 8 to be spaced upwardly from the liquid-absorbent structure under contraction of the barrier sheet elastic members 33, while the barrier sheet elastic members 33 are reliably anchored to the barrier sheet 8.
The second adhesion sites 42 comprise a pair of separate sites for each of the right and left barrier sheet elastic members 33. In this way, the total coating area can be reduced in comparison with the case in which these adhesion sites are respectively continuous, allowing the barrier sheet to be kept soft.

While the hot melt adhesive is coated over the entire areas of the first adhesion sites 40, 41 and second adhesion sites 42 and are intermittently coated in a wave pattern so as to leave clearance gaps in the third adhesion sites 43, in this embodiment, such intermittent coating in wave pattern with clearance gaps may be adopted for the first adhesion sites 40, 41 as well as for the second adhesion sites 42. It should be noted that, even in such cases, the hot melt adhesive must have the highest basis weight in the first adhesion sites 40, 41 and have a lower basis weight in the second adhesion sites 42 and the third adhesion sites 43, than in the first adhesion sites 40, 41.
The barrier sheet may be coated with the hot melt adhesive by the intermittent coating process, the multi-stripe coating process or the other processes such as the spraying process.

It should be noted that the respective basis weights of the first adhesion sites 40, 41, the second adhesion sites 42 and the third adhesion sites 43 are not limited to the values specified for this embodiment but may be appropriately selected depending on various factors such as a shrinkage degree of the barrier sheet elastic members 33 and a material quality of the barrier sheet 8.
While the third adhesion sites are formed on the first sheet 8a, and the first and second adhesion sites are formed on the second sheet 8b in this embodiment, it is possible to form the first and second adhesion sites on the first sheet 8a and to form the third adhesion sites on the second sheet 8b. It is also possible to form all of the adhesion sites on any one of the sheets 8a, 8b. The adhesion sites comprising the hot melt adhesive of a same type and processed by a same coating technique are preferably formed on one and same sheet for improvement of productive efficiency.

While the barrier sheet elastic members 33 continuously extend from the respective front end portions 24 to the respective rear end portions 25 of the barrier sheet 8 in this embodiment, it is possible to arrange these elastic members 33 discontinuosly, for example, in the middle portion 26 of the barrier sheet 8. In such an arrangement, the longitudinally opposite end segments of the elastic members may be anchored on the sheet by hot melt adhesive forming the first adhesion sites, while the remaining segments may be anchored on the sheet by the hot melt adhesive having a basis weight lower than the basis weight of the first adhesion sites to alleviate the skin irritation possibly experienced by the wearer.
While the basis weight of the hot melt adhesive is regulated to be lower in the third adhesion sites 43 than in the second adhesion sites 42 in this embodiment, it is possible to regulate the hot melt adhesive in the second and third adhesion sites 42, 43 to have the same basis weight so far as this basis weight is lower than the basis weight in the first adhesion sites 40, 41. Principally, the basis weight may be regulated to be low in an area as large as possible in order to alleviate a skin irritation possibly experienced by the wearer.
While the barrier sheet 8 is provided with the front and rear passage openings 27, 28 in this embodiment, it is possible to provide the barrier sheet 8 with any one of these passage openings. In this case, the barrier sheet 8 may be provided preferably with a rear passage opening 28 since the rear passage opening 28 serves to prevent body waste from coming in contact with the wearer's skin.

### <Example 2 - Second Embodiment>

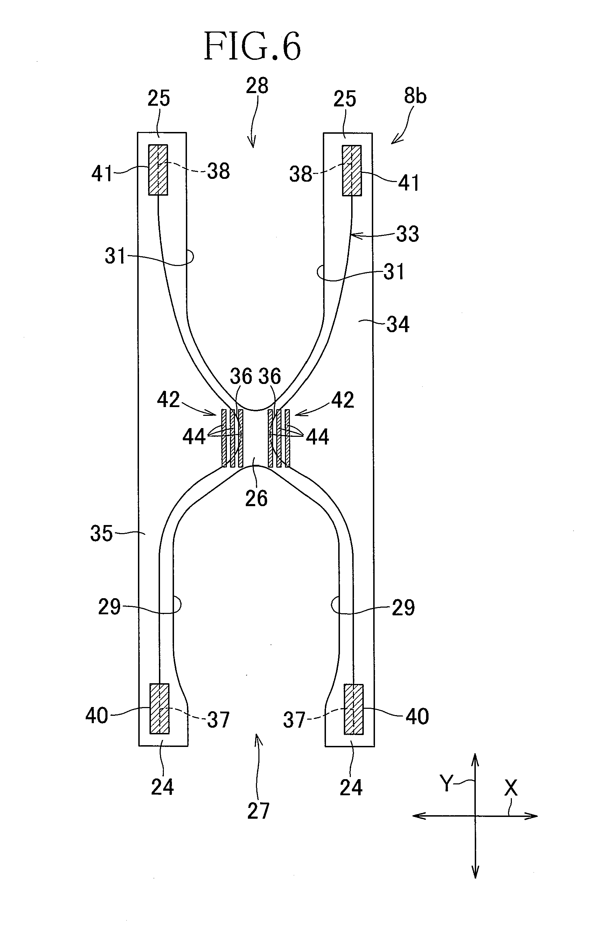

Fig. 6 is a scale-enlarged view illustrating the second sheet 8b of the barrier sheet 8 according to a second embodiment of the invention. This second embodiment is distinguished from the first embodiment in that each of the second adhesion sites 42 comprises a plurality of adhesion stripes 44 formed by coating the sheet 8b with hot melt adhesive. The first sheet 8a and the other components are similar to those in the first embodiment and detailed description thereof will be eliminated hereinafter.

Each of the right and left second adhesion sites 42, 42 comprises three adhesion stripes 44. These adhesion stripes 44 are formed by coating the second sheet 8b with the hot melt adhesive so as to extend in the longitudinal direction Y across the convex segments 36 of the barrier sheet elastic members 33. The second adhesion sites 42 are provided to prevent the convex segments 36 from elastically moving outward in the transverse direction and such function can be improved by arranging a plurality of the adhesion stripes 44 in the transverse direction X with a basis weight of the hot melt adhesive in each of the adhesion stripes 44 being reduced.
In addition, the basis weight of the hot melt adhesive can be reduced also merely because a plurality of the adhesion stripes are spaced one from another.

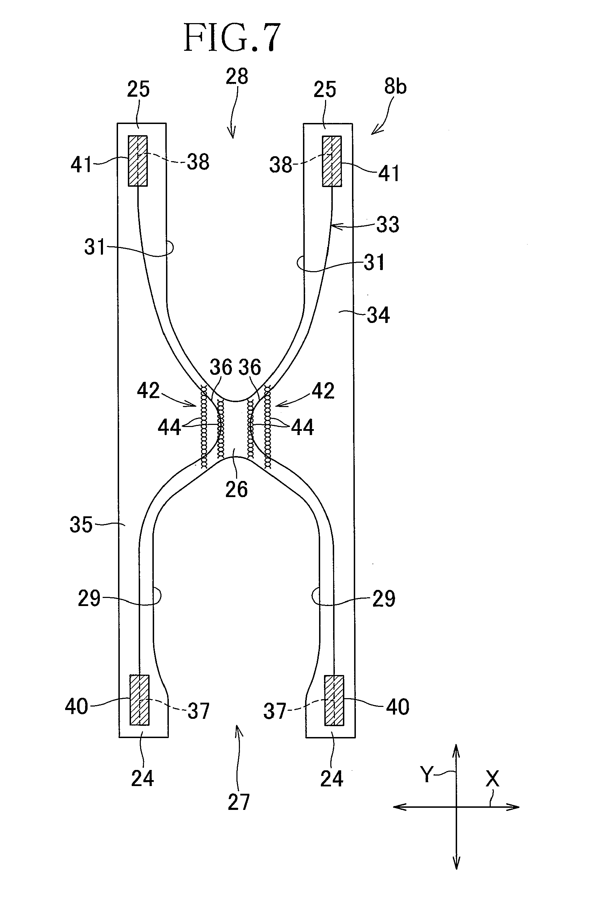

A plurality of the adhesion stripes are formed through the multi-stripe coating process so as to extend in parallel one to another in this second embodiment, it is possible to form them by a wave pattern formation coating process wherein each of adhesion stripes is obtained from a pair of wave patterns phase-shifted to overlap each other, and thereby a high adhesive strength thereof can be achieved.
While each of the second adhesion sites 42 comprises three adhesion stripes 44 in this embodiment, the number of the adhesion stripes 44 is not limited to this number. It is possible to form each of the second adhesion sites 42 from a pair of the adhesion stripes 44 as seen in Fig. 7. The number of adhesion stripes 44 forming each of the second adhesion sites 42 is optional so far as each of the second adhesion sites 42 comprises a plurality of the adhesion stripes 44 and effectively prevents the convex segments 36 from moving outward in the transverse direction X.

### <Example 3 - Third Embodiment>

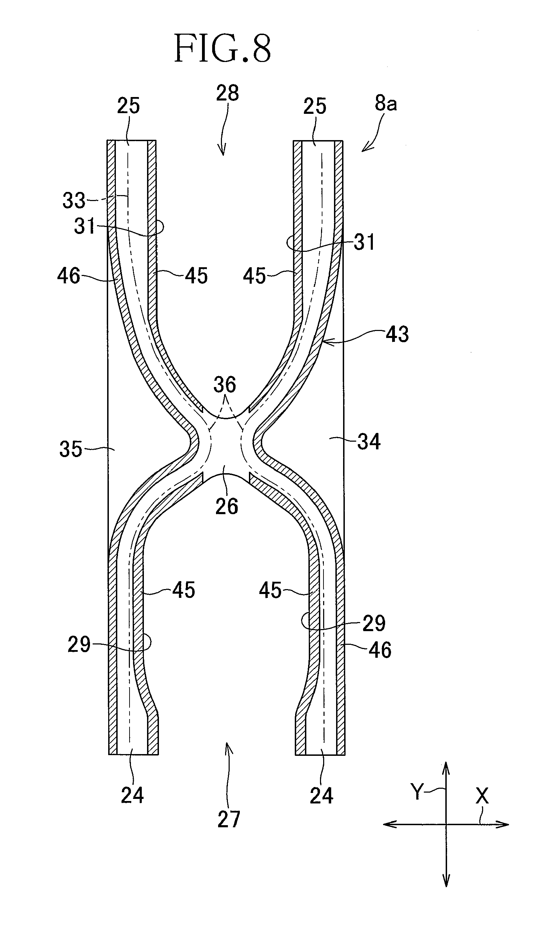

Fig. 8 is a scale-enlarged view illustrating the first sheet 8a of the barrier sheet 8 according to a third embodiment of the invention. The third embodiment is similar to the first embodiment except for the third adhesion site 43. The second sheet 8b and the other components are similar to those in the first embodiment and accordingly repetition of the associated detailed description thereof will be eliminated hereinafter.

According to the third embodiment, a pair of third adhesion sites 43 is formed to enclose the respective barrier sheet elastic members 33. Specifically, each of the third adhesion sites 43 comprises an inner adhesion site 45 extending on an inner side of the barrier sheet elastic member 33 as viewed in the transverse direction X, and an outer adhesion site 46 extending on an outer side of the barrier sheet elastic member 33. The inner adhesion site 45 extends along the inner side edges 29, 31 of the front and rear passage openings 27, 28, respectively, in the longitudinal direction Y from the longitudinally opposite ends of the sheet 8a and discontinued in the middle portion 26 in the longitudinal direction Y. In other words, any adhesion site serving to prevent the convex segment 36 of the barrier sheet 8 is absent on the inner side of the convex segment 36. However, the convex segment 36 is biased to move back outward in the transverse direction X and therefore it is unnecessary to provide any retaining means on the inner side of the convex segment 36.
Each of the outer adhesion sites 46 extends closely along the outer edges of the barrier sheet elastic members 33 as viewed in the transverse direction X across the lateral portions 34, 35 and the middle portion 26.

The third adhesion sites 43 formed around the barrier sheet elastic members 33 in this manner are effective to prevent the barrier sheet elastic members 33 from running off beyond the barrier sheet 8 and at the same time to bond the first and second sheets 8a, 8b together along the inner edges 29, 31 of the front and rear passage openings 27, 28. By coating the second sheet 8b with the hot melt adhesive only in the vicinity of the barrier sheet elastic members 33, the quantity of the hot melt adhesive coated on the second sheet 8b can be reduced in comparison with the case in which the second sheet 8b is coated over its entire area with the hot melt adhesive. Furthermore, movement of the barrier sheet elastic members 33 is not restrained by the hot melt adhesive in the third adhesion sites 43 and, in consequence, the barrier sheet elastic members 33 are allowed to stretch and contract uniformly.
By bisecting each of the inner adhesion sites 45 in the longitudinal direction Y, the quantity of the hot melt adhesive used in the middle portion 26 can be further reduced. In view of the fact that this middle portion 26 comes in contact with the wearer's crotch which is most sensitive to the skin irritation, the skin irritation can be effectively alleviated by reducing the quantity of the hot melt adhesive used in this portion.

### <Example 4 - Fourth Embodiment>

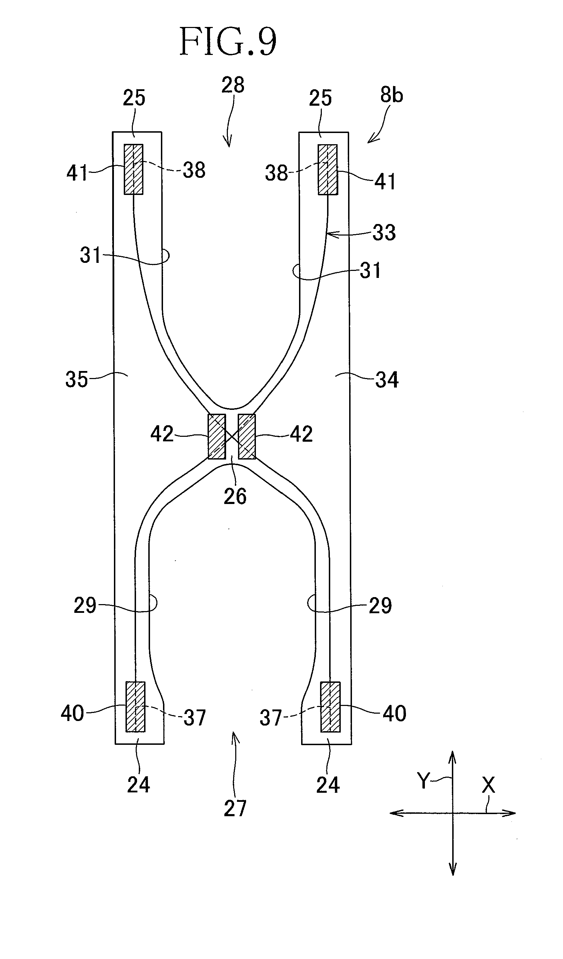

Fig. 9 is a scale-enlarged view illustrating the second sheet 8b of the barrier sheet 8 according to a fourth embodiment of the invention. The fourth embodiment is similar to the first embodiment except for the arrangement of the barrier sheet elastic members 33 and therefore repetition of the detailed description of the remaining components will be eliminated hereinafter.

Each of the barrier sheet elastic members 33 comprises a pair of rubber threads one of which extends from the front end portion 24 of the lateral portion 34 toward the rear end portion 25 of the lateral portion 35. The other rubber thread extends from the front end portion 24 of the lateral portion 35 toward the rear end portion 25 of the lateral portion 34.
The first adhesion sites 40, 41 are formed in the front and rear portions 37, 38 of the barrier sheet 33, the second adhesion sites 42 are formed in the vicinity of a cross-point of the barrier sheet elastic members 33 in the middle portion and the third adhesion sites 43 are formed in the portion other than these first and second adhesion sites 40, 41, 42. The first to third adhesion sites have basis weights similar to those in the first embodiment.

## Claims

1. An absorbent article (1) comprising a liquid-absorbent chassis (2) having a longitudinal direction, a transverse direction, a side facing the article wearer's body (100), a side facing the wearer's garment (101), a front waist region (3), a rear waist region (4), a crotch region (5) extending between said front and rear waist regions (3, 4) and a liquid-absorbent structure (7) provided in said crotch region (5), and a barrier sheet (8) provided on said side facing the article wearer's body (100) of said liquid-absorbent chassis (2) and adapted to be spaced from said liquid-absorbent chassis (2) wherein said barrier sheet (8) includes barrier sheet elastic members (33) attached under tension thereto and extending in a longitudinal direction, and wherein a space is formed between said barrier sheet (8) and said liquid-absorbent structure (7) as said liquid-absorbent structure (7) is bowed in the longitudinal direction, said absorbent article (1) being **characterized in that**:
said barrier sheet (8) comprises a pair of lateral portions (34, 35) opposed to and spaced from each other in said transverse direction, a middle portion (26) connecting said lateral portions (34, 35) to each other, front and rear passage openings (27, 28) defined by said lateral portions (34, 35) and said middle portion (26) through which body waste can be guided into said space and said lateral portions (34, 35) are attached at front and rear end portions (24, 25) thereof to said front and rear waist regions (3, 4);
said barrier sheet elastic members (33) are attached to said lateral portions (34, 35) and extend along said passage openings; and
said barrier sheet (8) has first adhesion sites (40, 41) at which front and rear end portions (24, 25) of said barrier sheet elastic members (33) are anchored on the barrier sheet (8) and middle adhesion sites (42) at which middle segments of said barrier sheet elastic members (33) extending between said first adhesion sites (40, 41) are anchored to the barrier sheet wherein a basis weight of adhesive is lower in said middle adhesion sites (42) than in said first adhesion sites (40, 41).

2. The absorbent article (1) as claimed in Claim 1, wherein:
said barrier sheet elastic members (33) extend from said front end portions of said lateral portions (34, 35) to said rear end portions and describe convex segments bowing toward a longitudinal center line bisecting a size of the barrier sheet in said transverse direction; and
said middle adhesion sites (42) comprising second adhesion sites at which said convex segments are anchored to the barrier sheet (8) and third adhesion sites (43) at which segments of said barrier sheet elastic members (33) extending between said first adhesion sites (40, 41) and said second adhesion sites (42) are anchored to the barrier sheet (8).

3. The absorbent article (1) as claimed in Claim 1, wherein:
each of said barrier sheet elastic members (33) extends from said front end portion of one of said lateral portions (34, 35) to said rear end portion of the other of said lateral portions (34, 35) so that said barrier sheet elastic members (33) intersect with each other in said middle portion (26); and
wherein said middle portion (26) includes second adhesion sites (42) at which said intersecting segments are anchored to said barrier sheet (8) and a third adhesion sites (43) at which segments of said barrier sheet elastic members (33) extending between said first adhesion sites (40, 41) and said second adhesion sites (42) are anchored on said barrier sheet (8).

4. The absorbent article (1) as claimed in Claim 2 or 3, wherein the adhesive in said third adhesion sites (43) has a basis weight lower than a basis weight of the adhesive in said second adhesion sites (42).

5. The absorbent article (1) as claimed in any one of Claims 2 to 4, wherein the barrier sheet (8) is coated at said first adhesion sites (40, 41) and said second adhesion sites (42) over the entire area thereof with said adhesive and intermittently coated with said adhesive at said third adhesion sites (43).
